# EUROPEAN PATENT SPECIFICATION

(11) **EP 3 714 849 B1**
(45) Date of publication and mention of the grant of the patent: **11.11.2026**
(21) Application number: 20166609.6
(22) Date of filing: 30.03.2020
(51) Int. Cl.: A61F 9/02

(54) **GOGGLE WITH STRAP RETENTION SYSTEMS AND METHOD FOR ITS MANUFACTURE**
SCHUTZBRILLE MIT BANDRÜCKHALTESYSTEMEN UND VERFAHREN ZU DEREN HERSTELLUNG
MASQUE DE PROTECTION COMPRENANT DES SYSTÈMES DE RÉTENTION DE SANGLE ET SON PROCÉDÉ DE FABRICATION

(30) Priority: 29.03.2019 US 201962826889 P; 19.03.2020 US 202016824525
(43) Date of publication of application: 30.09.2020
(73) Proprietor: 100% Speedlab, LLC, San Diego, CA 92123 (US)
(72) Inventor: YOUNG, Michael D., San Diego, CA California 92123 (US)
(74) Representative: Murgitroyd & Company

(56) References cited:
- WO-A1-2017/189130
- DE-A1- 3 503 393
- US-A- 5 809 580
- US-A1- 2011 197 346

## Description

### TECHNICAL FIELD

One or more embodiments relate generally to goggles and, more particularly, to systems and techniques for coupling a goggle strap to a goggle frame.

### BACKGROUND

Sport goggles are worn by users for various sports or activities, such as motorsports, powersports, snowsports, watersports, biking, or the like, to protect wearers' eyes. A sport goggle may be held to the wearer's eyes through a strap. During activities, the goggle may receive forces from, for example, impacts that may stretch or otherwise impart a pulling force on the strap. Additionally, a wearer may remove the goggle by pulling on a frame of the goggle and, thus, stretch the strap. Accordingly, the strap must be secured to the goggle frame in a robust manner to avoid failure of the strap, frame, and/or mechanisms for coupling the strap and frame to each other when the strap is stretched.

Furthermore, sport goggles may be bulky. A bulky goggle may be heavy, may result in additional unwanted impacts, may be harder to wear, may reduce the field of vision of the wearer, and/or may be more difficult to handle in other ways.
DE3503393 discloses a goggle having a box-like, injection-moulded plastic frame on the front side of which a pane is arranged and the back side of which has contact surfaces formed to correspond to the facial contour, the frame comprising fixing means for a lengthwise-adjustable retaining strap to go around the head of the wearer, in order to simplify production and assembly and to achieve easy adjustability of the retaining strap, as well as to permit venting of the interior space in the worn state.
WO 2017/189130 discloses a goggle including a frame, a lens, and a locking system. The frame has a first lateral portion, an opposing second lateral portion, an upper portion, and a lower portion defining a cavity therebetween. The lens is disposed across the cavity. The locking system is configured to selectively secure the lens to the frame. The locking system includes a first locking member releasably coupled to the first lateral portion of the frame and a second locking member releasably coupled to the opposing second lateral portion of the frame. There remains a need for improved sport goggles.

### SUMMARY

According to the present invention, there are provided a goggle according to claim 1 and a method according to claim 8.

### BRIEF DESCRIPTION OF THE DRAWINGS

Fig. 1 shows a perspective view of a goggle, in accordance with an embodiment of the invention.
Fig. 2 shows an exploded view of portions of the goggle of Fig. 1, in accordance with an embodiment of the invention.
Fig. 3 shows a perspective view of a portion of the goggle of Fig. 1, in accordance with an embodiment of the invention.
Figs. 4 shows a view of a strap and cage configured to be coupled to a goggle frame not according to the invention.
Fig. 5 shows a view of a strap and cage configured to be coupled to a goggle frame, in accordance with an embodiment of the invention.
Fig. 6 shows a top view of the goggle of Fig. 1, in accordance with an embodiment of the invention.
Figs. 7 and 8 show cutaway views of the goggle of Fig. 6, in accordance with embodiments of the invention.
Fig. 9 shows a perspective view of a cage useful for understanding the invention.
Fig. 10 shows a flowchart detailing a method of making the goggle of Fig. 1, in accordance with an embodiment of the invention.
Fig. 11 shows a flowchart detailing a method of making and using the goggle of Fig. 1, in accordance with an embodiment of the invention.

Embodiments of the invention and their advantages are best understood by referring to the detailed description that follows. It should be appreciated that like reference numerals are used to identify like elements illustrated in one or more of the Figures.

### DETAILED DESCRIPTION

A goggle that includes a cage to couple the strap of the goggle to the goggle frame is described herein. The systems and techniques described herein allow a strap to be coupled to a goggle frame through a cage that offers a smaller form factor and a stronger connection. Thus, the cage allows for improved strap to goggle frame coupling.

Typically, the strap of a sports goggle is directly coupled to the goggle frame. That is, the goggle frame, which may be a plastic or other type of molded frame, has attachment points on the periphery of the goggle frame configured to receive the strap. As the goggle frame is made of plastic, such attachment points may need to be reinforced, leading to a bulkier goggle frame. Especially as the attachment points are typically on the sides of the goggle frame, the wearer's peripheral vision may be impacted.

A goggle is disclosed in accordance with various embodiments. In a certain embodiment, the goggle includes a goggle frame, a goggle lens, a cage, and an elastic band. The goggle frame includes an outrigger disposed on an end of the goggle frame. The goggle lens is disposed within the goggle frame. The outrigger includes an outrigger opening that is configured to receive the cage. The cage include a first opening configured to receive an elastic band and a protrusion disposed within the first opening and configured to couple to the elastic band to hold the elastic band within the first opening.

The cage may be, in certain embodiments, made from metal or another material stronger than the material of the goggle frame. Thus, the cage may be configured to receive a greater force component than typical goggle frame based attachment techniques before failure of the coupling. Additionally, the cage may be made to be less bulky than that of a traditional goggle frame attachment technique. Features within the opening of the cage where the strap is disposed within may aid in the cage holding the elastic band. Features on the outside of the cage may aid in attaching the cage to the opening of the goggle frame and/or outrigger.

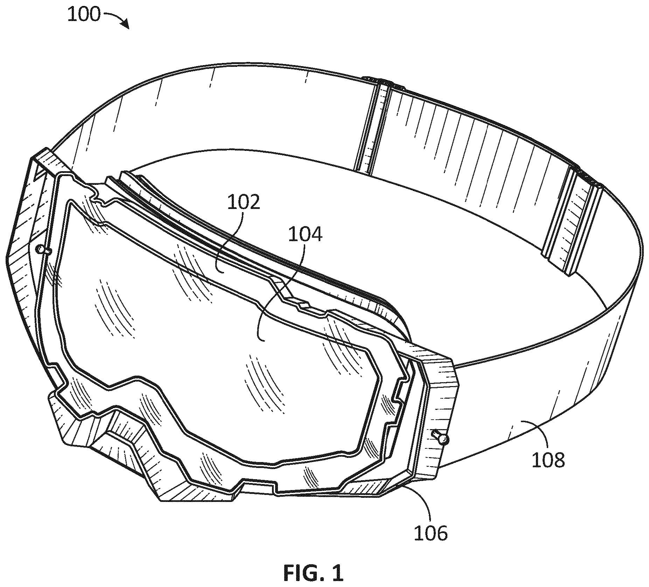

Fig. 1 shows a perspective view of a goggle, in accordance with an embodiment of the invention. Fig. 1 illustrates goggle 100 that includes goggle frame 102, goggle lens 104, outrigger 106, and elastic band 108.

The goggle frame 102 is configured to receive the goggle lens 104. The goggle frame 102 may be made of any appropriate material, such as one or more of plastics, composites, metals, or other materials. The goggle frame 102 may be formed by, for example, molding, casting, machining, and/or coupling together (e.g., gluing or mechanically fastening) of one or more multiple components.

The goggle frame 102 includes the outrigger 106. The outrigger 106 may, in certain embodiments, be an extension of the goggle frame 102. In other embodiments, the outrigger 106 may be separate from the goggle frame 102. Thus, for example, the outrigger 106 may be formed separate from the goggle frame 102 and coupled to the goggle frame 102 (e.g., mechanically, adhesively, or through another technique). The outriggers are disposed on lateral ends of the goggle frame. Thus, a first outrigger is disposed on a first end of the goggle frame 102 (e.g., the left side of the goggle frame 102) and a second outrigger is disposed on a second end of the goggle frame 102 (e.g., the right side of the goggle frame 102). Each outrigger includes an opening that receives a corresponding cage.

In certain embodiments, the outrigger 106 may define at least a portion of an opening of the goggle frame 102 configured to receive the goggle lens 104.

Elastic band 108 is coupled to the goggle frame 102. The elastic band 108 may be, for example, a strap that is configured to, when the goggle 100 is worn by a wearer, hold the goggle 100 to the face of the wearer. As such, the elastic band 108 may be configured to be stretched to conform with the wearer's head, to allow for the goggle 100 to be disposed on the wearer's head, to allow for the goggle 100 to be removed from the wearer's head, and/or for other reasons. The elastic band 108 may be adjustable in length to accommodate a variety of head shapes. Additionally, as described herein, the elastic band 108 is coupled to a cage, and the cage is then accordingly coupled to the outrigger 106.

In certain embodiments, the goggle frame 102 may include an opening configured to receive the goggle lens 104. In certain such embodiments, the goggle lens 104 may be configured to be disposed within and to decouple from the goggle frame 102 (e.g., to allow for replacement of the goggle lens 104). The goggle lens 104 may be any appropriate goggle lens that allows a wearer to view objects on the other side of the goggle lens 104. The goggle lens 104 may, thus, be an opaque lens configured to allow viewing through the lens by the wearer while protecting the wearer's eyes.

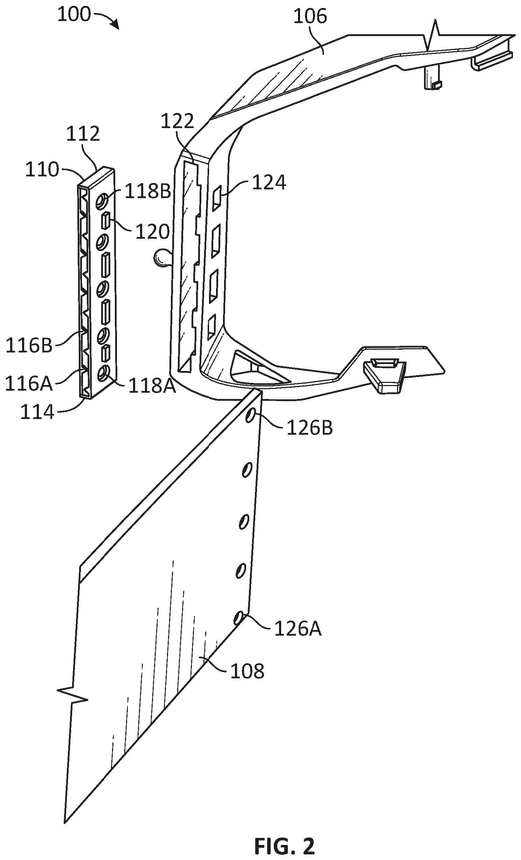

Fig. 2 shows an exploded view of portions of the goggle of Fig. 1, in accordance with an embodiment of the invention. Fig. 2 illustrates an exploded view of the goggle 100 of Fig. 1 that includes the outrigger 106, the elastic band 108, and a cage 110. In certain embodiments, the outrigger 106 may be a portion of the goggle frame 102.

As shown in Fig. 2, the elastic band 108 may be coupled to the cage 110. The cage may then be disposed within an opening 122 of the outrigger 106. Thus, the elastic band 108 may be coupled to the outrigger 106 and, accordingly, the goggle frame, through the cage 110. Such a configuration may allow for the elastic band 108 to have a stronger and more compact coupling to the outrigger 106 and/or the goggle frame 102.

The cage 110 may be, in certain embodiments, made from metal or another material stronger than the material of the goggle frame 102 and/or the outrigger 106 (e.g., a modulus of elasticity of the material of cage 110 may be higher than the modulus of elasticity of the material of the goggle frame 102 and/or the outrigger 106). Thus, coupling the elastic band 108 to the cage 110 and then disposing the cage 110 within the opening 114 may lead to a stronger connection than typical techniques that attach the elastic band directly to the goggle frame and/or the outrigger.

Furthermore, the cage 110 may be more compact (e.g., narrower) than a typical connection while maintaining or improving strength. Thus, the outrigger 106 and/or the goggle frame 102 may be of a smaller size. As, typically, the elastic band 108 is coupled to the sides of the goggle 100 (e.g., disposed to the left and right of the face of the wearer), such a configuration may lead to a more compact goggle frame 102 and/or more compact outrigger 106 that can improve the wearer's field of vision.

The cage 110 includes features for coupling to the elastic band 108 and/or coupling to the outrigger 106. The cage 110 includes a cage body 112. The cage body 112 includes an opening 114. The opening 114 may be substantially rectangular in cross section to receive the elastic band 108. The opening 114 may be of a height and width configured to accommodate the elastic band 108. Thus, the width of the opening 114 may be wider than the thickness of the elastic band 108. The width of the opening 114 may also accordingly be wider than the width of the elastic band 108.

The cage 110 includes features to hold the elastic band 108 when the elastic band 108 is disposed within the opening 114. The cage 110 includes protrusions 116 that aid in holding the elastic band 108 within the opening 114. The protrusions 116 may be, for example, teeth with triangular, square, or other geometries, depressions, adhesives, rods, and/or other types of protrusions or features that may aid in retaining the elastic band 108 within the opening 114. The protrusions 116 are disposed within a portion of the opening 114. While the embodiment shown in Fig. 2 includes multiple triangular teeth shaped protrusions 116A-B and others, other embodiments may include protrusions and/or retaining features of other geometries and/or may include more or fewer protrusions.

The elastic band 108 may additionally include openings 126A, 126B, and others. Various embodiments may include any number of such openings. The cage may include corresponding openings 118A, 118B, and others. The openings 118A and 118B may be configured to correspond to the openings 126A and 126B so that, when the elastic band 108 is disposed within the opening 114, the opening 118A is aligned with the opening 126A, the opening 118B is aligned with the opening 126B, and so on. When the openings 126A and 126B are aligned with the openings 118A and 118B, respectively, fasteners may be disposed through each pair of openings to aid in holding the elastic band 108 within the opening 114 of the cage 110.

The cage 110 additionally includes detent 120 and other features disposed on an exterior portion of the cage 110. Such features are configured to interface with corresponding feature 124 within the opening 122. The detent 120 may be, for example, one or more raised surfaces (e.g., protrusion) or depressions, fasteners, adhesives, or other features so that, when the elastic band 108 is pulled, the detent 120 and the corresponding feature 124 may form a mechanical interface (e.g., may include contacting walls) to hold the cage 110 within the opening 122. The corresponding feature 124 within the opening 122 may include depressions to correspond to raised surfaces, raised surfaces to correspond to depressions, and/or corresponding fasteners or features. Thus, the elastic band 108 may be more securely disposed within the opening 114.

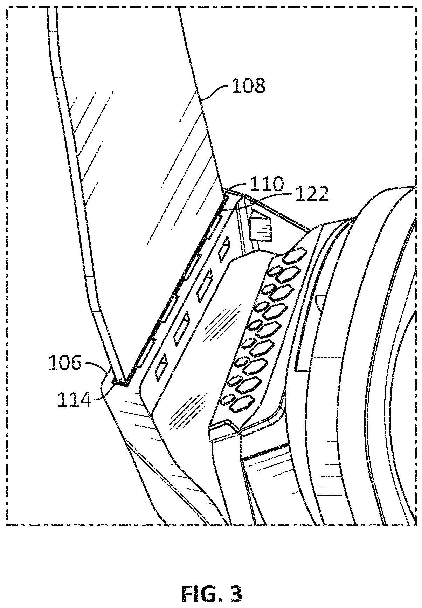

Fig. 3 shows a perspective view of a portion of the goggle of Fig. 1, in accordance with an embodiment of the invention. Fig. 3 illustrates the cage 110 disposed within the opening 122 of the outrigger 106. As such, the cage 110 is coupled to the elastic band 108 and is disposed within the opening 122. The cage 110 is held within the opening 122 through the features 124 as described herein. The elastic band 108 may also accordingly be held within the opening 114.

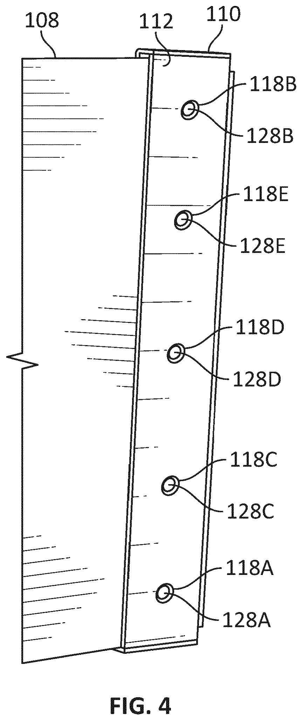

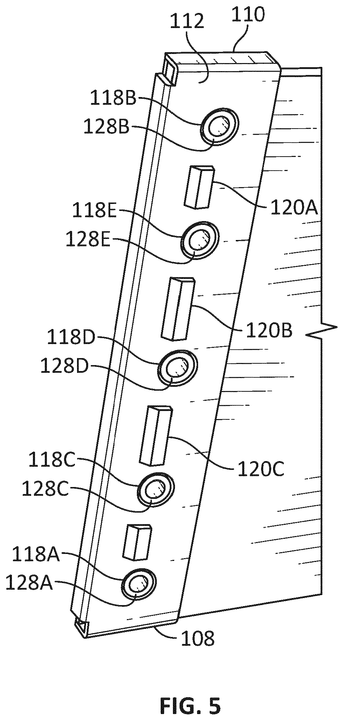

Fig. 4 shows the elastic band 108 coupled to the cage 110 not according to the invention. As shown, the cage 110 includes openings 118A-E. The elastic band 108 may include corresponding openings (e.g., openings 126A-E; not shown in Figs. 4 and 5, but shown in Fig. 2). The openings 118A-E may be aligned with the openings 126A-E and fasteners 128A-E may be disposed through the openings 118A-E and 126A-E. As such, the fasteners 128A-E may aid in holding the elastic band 108 within the opening 114 of the cage 110.

In certain embodiments, the fasteners 128A-E may be, for example, a rivet, a mushroom cap, a bolt and/or a nut, a snap, a protrusion of the cage 110, a dowel, adhesive, teeth, and/or other type of feature configured to further secure the elastic band 108 within the opening 114 of the cage 110. In certain such embodiments, the openings 118A-E may be substantially (e.g., +/-20% in variance) evenly spaced on the cage 110. Thus, the distance between each of the openings 118A-E may be substantially similar. Furthermore, at least two of the openings 118A-E (e.g., the openings 118A and 118B) may be disposed on opposing sides of the cage 110. Disposing the openings in such a manner allows for more even distribution of stresses (e.g., resulting from pulling on the elastic band 108) within portions of the elastic band 108 and/or the cage 110, further increasing strength.

Fig. 5 illustrates detents 120A-E on an exterior surface of the cage 110 according to an embodiment of the invention. The detents 120A-E may be, for example, one or more raised surfaces (e.g., protrusion) or depressions, fasteners, adhesives, or other features configured to interface with corresponding features the opening 122 of the outrigger 106 and/or goggle frame 102. Such corresponding features may include depressions to correspond to raised surfaces, raised surfaces to correspond to depressions, and/or corresponding fasteners or other features. Detents 120A-E allow for the cage 110 to be more securely disposed within the opening 114 and, thus, increase the strength of the coupling of the elastic band 108 to the goggle frame 102 and/or the outrigger 106.

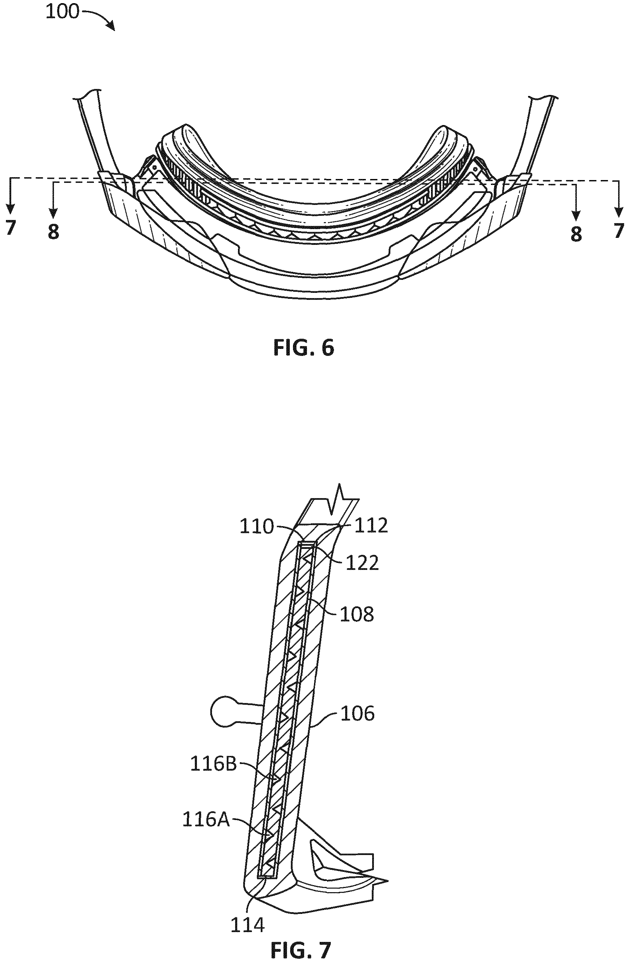

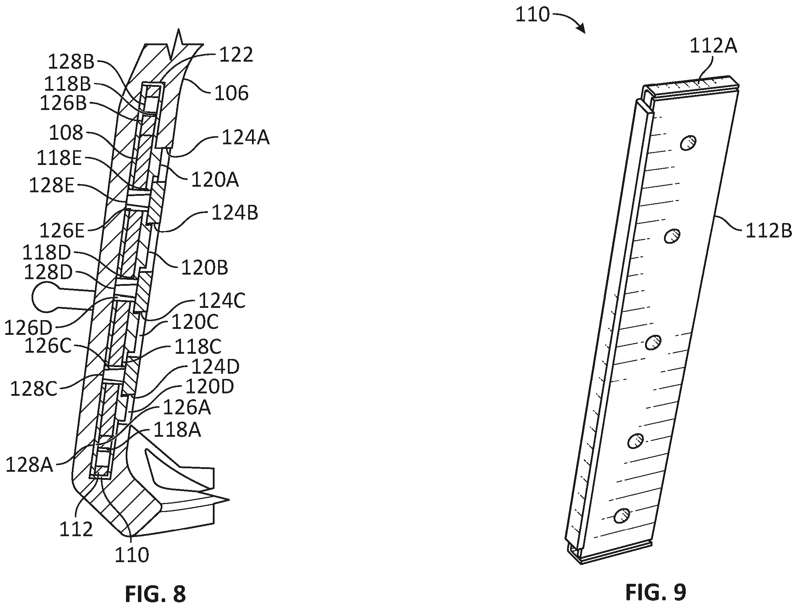

Fig. 6 shows a top view of the goggle of Fig. 1, in accordance with an embodiment of the invention. Fig. 6 is a top view of the goggle 100. Fig. 6 shows planes 7-7 and 8-8. Figs. 7 and 8 show cutaway views of the goggle of Fig. 6, in accordance with embodiments of the invention. The cutaway view of Fig. 7 is taken along plane 7-7. The cutaway view of Fig. 8 is taken along plane 8-8.

The cutaway view of Fig. 7 illustrates the cage 110 disposed within the opening 122 of the outrigger 106. As shown, the cage 110 may be substantially rectangular in cross-sectional shape and the opening 122 may include a shape that corresponds to the cross-sectional shape of the cage 110 to hold the cage 110 within the opening 122. The cutaway view of Fig. 7 further illustrates the elastic band 108 disposed within the opening 114 of the cage 110. While disposed within the opening 114, the protrusions 116A and 116B, as well as other protrusions illustrated, may grip the elastic band 108 to aid in holding the elastic band 108 within the opening 114. The protrusions may, for example, dig into the side of the elastic band 108 to grip the elastic band 108.

The cutaway view of Fig. 8 illustrates that the detents 120A-D of the cage 110 are disposed within the features 124A-D. While certain embodiments of the features 124A-D may be depressions within the goggle frame 102 and/or the outrigger 106, the embodiment shown in Fig. 8 illustrates that the features 124A-D may also be openings within the goggle frame 102 and/or the outrigger 106. The detents 120A-D may be disposed within at least a portion of the features 124A-D, respectively. The features 124A-D may include one or more surfaces to prevent the detents 120A-D (and, thus, the cage 110) from moving in one or more directions while disposed within the features 124A-D. Certain embodiments may constrain different directions of movement. Such directions may include a direction that prevents rearward movement of the cage 110 due to, for example, a pulling force exerted on the elastic band 108.

Additionally, as shown in Fig. 8, the elastic band 108 may be coupled to the cage 110 through the fasteners 128A-E. Portions of the fasteners 128A-E may be disposed within the openings 118A-E of the cage 110 and openings 126A-E of the elastic band 108 to couple the elastic band 108 to the cage 110.

Fig. 9 shows a perspective view of a cage 110 useful for understanding the invention. As shown in Fig. 9, the cage 110 is made of a plurality of separate portions. That is, cage 110 includes cage bodies 112A and 112B. Each of cage bodies 112A and 112B may be, for example, a part manufactured from folded sheetmetal, stamped sheetmetal, machined stock, cast metal, molded composites, or other techniques. In certain embodiments, the cage bodies 112A and 112B may be made from separate techniques.

The separate cage bodies 112A and 112B are coupled together (e.g., friction fit, mechanically coupled, adhesively coupled, or coupled through other techniques) to form the cage 110. The elastic band 108 is disposed between the cage bodies 112A and 112B and the cage bodies 112A and 112B are accordingly coupled together to form the cage 110. The cage 110 may then be disposed within the opening 122.

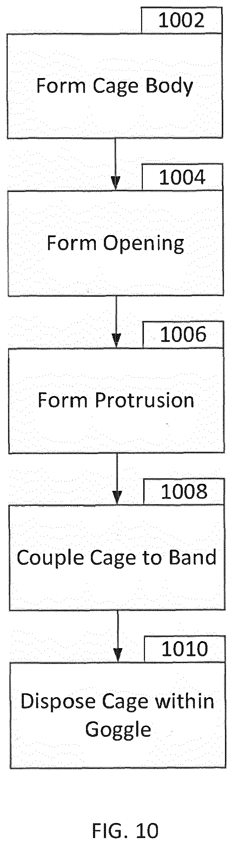

Fig. 10 shows a flowchart detailing a method of making the goggle of Fig. 1, in accordance with an embodiment of the invention. In block 1002, the cage body of the cage may be formed. The cage body may, in certain embodiments, be a metal cage body. The cage body may be formed from bent sheetmetal (e.g., through one or more bending operations performed on a flat pattern), machined from stock, cast, molded, extruded, or otherwise appropriately formed.

After the cage body has been formed in block 1002, the opening may be formed in block 1004. In certain embodiments, the opening may be formed when the cage body is formed. Thus, for example, in embodiments where the body is formed through bent sheetmetal, bending of the sheetmetal may accordingly form the opening. Furthermore, if the cage body is cast, the casting operation may form the opening at the same time as the cage body. In other embodiments, such as embodiments where the cage is machined or cast, the opening may be formed in a separate operation. Thus, for example, the opening may be machined into the cage body.

After the opening has been formed, any protrusions within the opening may be formed in block 1006. For example, in embodiments where the opening and/or the cage body is formed from bent sheetmetal, additional portions of the flat pattern may be bent to form the protrusions. Alternatively or additionally, the protrusions may be machined when the opening is machined. In other embodiments, the protrusions may be formed as a separate component and may be disposed within the opening and coupled to the cage body (e.g., through fasteners, adhesives, welding, or other techniques).

In block 1008, the elastic band may be disposed within the opening to couple the elastic band to the cage. In certain embodiments, the elastic band may be inserted into the opening and held by the protrusions. In other embodiments, the elastic band may first be inserted onto a flat pattern or a partially formed opening. The opening may then be formed around the elastic band. Thus, for example, the elastic band may be disposed within a partially formed opening and the opening may then be finished around the elastic band by, for example, performing the remaining bending steps to form the opening. In such an example, the protrusions may be formed (e.g., bend) before the opening is fully formed. Thus, when the opening is fully bent, the protrusions may accordingly grasp the elastic band in a manner to retain the elastic band.

In block 1010, the cage is disposed within the outrigger. The cage may, thus, be inserted into an opening of the outrigger. The cage is held within the opening through features on the exterior of the cage and/or the opening of the outrigger. In certain embodiments, the cage may be held within the opening by adhesives or other coatings. Thus, for example, the opening and/or the cage may include a compressible coating that is configured to be deformed when the cage is disposed within the opening, further aiding in preventing the cage from separating from the opening.

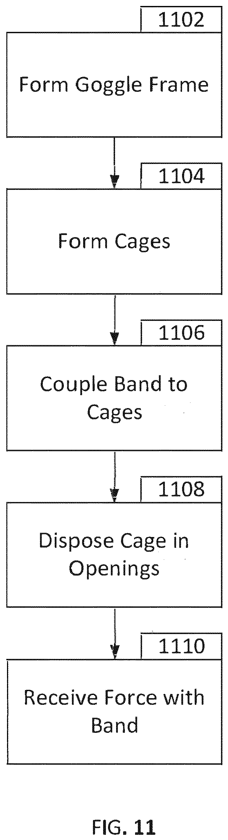

Fig. 11 shows a flowchart detailing a method of making and using the goggle of Fig. 1, in accordance with an embodiment of the invention. In block 1102, the goggle frame may be formed, as described herein. In certain embodiments, the goggle frame and the outriggers may be formed separately and the outriggers may be accordingly coupled to the goggle frame.

In block 1104, the cages may be formed, as described herein. The elastic band is coupled to the cages in block 1106 by, for example, inserting the elastic band into the opening of the cages. The elastic band includes a first end and a second end and the first end is inserted into a first cage and the second end is inserted into the second cage. Additionally, each cage may be composed of a plurality of portions. As such, the elastic band may be disposed within the plurality of portions and the portions may then be coupled together to form the cage and hold the end of the elastic band within the cage.

In block 1108, the cages may be disposed within the openings of the the outriggers, as described herein, to complete the goggle. The goggle may then be used in block 1110. In block 1110, a force may be received by the elastic band. The elastic band may stretch and exert a force on the coupling between the elastic band and the cage. Features of the cage (e.g., the protrusions) may hold the elastic band within the cage. The force may then be transferred to the opening through the cage. The cage may be held within the opening (e.g., through features of the cage such as the detents). As such, despite a force being exerted on the elastic band, the elastic band may remain coupled to the goggle frame through the cage.

It is appreciated that the embodiments described herein are for exemplary purposes only. Thus, various embodiments may include some or all of the features described herein. Furthermore, the steps described in Figs. 10 and/or 11 may, in other embodiments, be performed in an order different from the order described in Figs. 10 and/or 11.

## Claims

1. A goggle (100) comprising:
an elastic band (108) having a first end held by one or more protrusions (116) within a band opening (114) of a body (112) of a first coupling piece (110) and a second end held by one or more protrusions (116) within a band opening (114) of a body (112) of a second coupling piece (110); and
a goggle frame (102) having a first outrigger (106) and a second outrigger (106) disposed on respective lateral ends of the frame (102), wherein the first and second outriggers (106) each have a respective outrigger opening (122) configured to receive one of the first and second coupling pieces (110);
wherein each of the first and second coupling pieces (110) has one or more detents (120) and each of the first and second outrigger openings (122) has one or more corresponding features (124) configured to interface with the one or more detents (120);
wherein each of the first and second coupling pieces (110) is adapted to be inserted into a respective one of the outrigger openings (122) while holding the elastic band (108), and to be held in place by the one or more detents (120), such that the elastic band (108) is coupled to the goggle frame (102); and
wherein the body (112) of the first coupling piece (110) comprises a first cage body (112A) and a second cage body (112B) coupled to the first cage body (112A), and the elastic band (108) is disposed between the first and second cage bodies (112A, 112B).

2. The goggle (100) of claim 1, wherein each of the first and second coupling pieces (110) further comprises a first fastener opening (118A-118E), wherein each first fastener opening (118A-118E) is configured to receive a first fastener (128A-128E).

3. The goggle (100) of claim 2, wherein each of the first and second coupling pieces (110) further comprises a second fastener opening (118A-118E), wherein each second fastener opening (118A-E) is configured to receive a second fastener (128A-128E), and wherein the first and second fastener openings are disposed on opposing sides of the coupling piece (110).

4. The goggle (100) of claim 1, wherein the one or more detents (120) are protrusions and the corresponding features (124) are depressions.

5. The goggle (100) of claim 1, wherein the body of the first coupling piece is metal, wherein the one or more protrusions of the first coupling piece are substantially triangular shaped teeth, wherein the band opening of the first coupling piece comprises a substantially rectangular cross section, and wherein the teeth protrude into the substantially rectangular cross section.

6. The goggle (100) of claim 1, wherein the first and second cage bodies (112A, 112B) each comprise folded sheet metal.

7. The goggle (100) of claim 1, further comprising:
a goggle lens (104) disposed within the goggle frame (102).

8. A method of manufacturing the goggle (100) of claim 1, the method comprising:
forming the goggle frame (102);
forming the first coupling piece (110) and the second coupling piece (110), wherein forming the first coupling piece (110) includes forming the first cage body (112A) and the second cage body (1128) and coupling the first cage body (112A) to the second cage body (112B);
coupling the first coupling piece (110) to the first end of the elastic band (108), and coupling the second coupling piece (110) to the second end of the elastic band (108);
inserting the first coupling piece (110) and the first end of the elastic band (108) within the first outrigger opening (122); and
inserting the second coupling piece (110) and the second end of the elastic band (108) within the second outrigger opening (122).

9. The method of claim 8, wherein the first outrigger (106) and the second outrigger (106) are formed with the goggle frame (102).

10. The method of claim 8, further comprising:
forming the first outrigger (106);
forming the second outrigger (106); and
coupling the first outrigger (106) and the second outrigger (106) to the goggle frame (102).

11. The method of claim 8, wherein coupling the first coupling piece (110) to the first end of the elastic band (108) comprises disposing the first end of the elastic band (108) within the band opening (114) of the first coupling piece (110).

12. The method of claim 8,
wherein forming the body (112) of the first coupling piece (110) comprises forming the one or more protrusions (116) within the band opening (114).

13. The method of claim 12, wherein the body (112) and the one or more protrusions (116) of the first coupling piece (110) are formed by machining a metal stock or three-dimensional printing.

14. The method of claim 12, wherein forming the body (112) of the first coupling piece (110) comprises forming the body (112) from sheet metal to define the band opening (114), and wherein forming the one or more protrusions (116) comprises bending the protrusions (116) into the band opening (114).

## Patentansprüche

1. Eine Schutzbrille (100), die Folgendes beinhaltet:
ein elastisches Band (108) mit einem ersten Ende, das von einem oder mehreren Vorsprüngen (116) innerhalb einer Bandöffnung (114) eines Körpers (112) eines ersten Kopplungsstücks (110) gehalten wird, und einem zweiten Ende, das von einem oder mehreren Vorsprüngen (116) innerhalb einer Bandöffnung (114) eines Körpers (112) eines zweiten Kopplungsstücks (110) gehalten wird; und
einen Schutzbrillenrahmen (102) mit einem ersten Ausleger (106) und einem zweiten Ausleger (106), die an jeweiligen seitlichen Enden des Rahmens (102) angeordnet sind, wobei der erste und der zweite Ausleger (106) jeweils eine jeweilige Auslegeröffnung (122) aufweisen, die konfiguriert ist, um eines von dem ersten und dem zweiten Kopplungsstück (110) aufzunehmen;
wobei jedes von dem ersten und dem zweiten Kopplungsstück (110) eine oder mehrere Feststellvorrichtungen (120) aufweist und jede von der ersten und der zweiten Auslegeröffnung (122) ein oder mehrere entsprechende Merkmale (124) aufweist, die konfiguriert sind, um sich mit der einen oder den mehreren Feststellvorrichtungen (120) zu verbinden;
wobei jedes von dem ersten und dem zweiten Kopplungsstück (110) angepasst ist, um in eine jeweilige der Auslegeröffnungen (122) eingeführt zu werden, während es das elastische Band (108) hält, und um von der einen oder den mehreren Feststellvorrichtungen (120) an Ort und Stelle gehalten zu werden, sodass das elastische Band (108) an den Schutzbrillenrahmen (102) gekoppelt ist; und
wobei der Körper (112) des ersten Kopplungsstücks (110) einen ersten Gehäusekörper (112A) und einen an den ersten Gehäusekörper (112A) gekoppelten zweiten Gehäusekörper (112B) beinhaltet und das elastische Band (108) zwischen dem ersten und dem zweiten Gehäusekörper (112A, 112B) angeordnet ist.

2. Schutzbrille (100) gemäß Anspruch 1, wobei jedes von dem ersten und dem zweiten Kopplungsstück (110) ferner eine erste Befestigungselementöffnung (118A-118E) beinhaltet, wobei jede erste Befestigungselementöffnung (118A-118E) konfiguriert ist, um ein erstes Befestigungselement (128A-128E) aufzunehmen.

3. Schutzbrille (100) gemäß Anspruch 2, wobei jedes von dem ersten und dem zweiten Kopplungsstück (110) ferner eine zweite Befestigungselementöffnung (118A-118E) beinhaltet, wobei jede zweite Befestigungselementöffnung (118A-E) konfiguriert ist, um ein zweites Befestigungselement (128A-128E) aufzunehmen, und wobei die erste und die zweite Befestigungselementöffnung auf gegenüberliegenden Seiten des Kopplungsstücks (110) angeordnet sind.

4. Schutzbrille (100) gemäß Anspruch 1, wobei die eine oder die mehreren Feststellvorrichtungen (120) Vorsprünge sind und die entsprechenden Merkmale (124) Vertiefungen sind.

5. Schutzbrille (100) gemäß Anspruch 1, wobei der Körper des ersten Kopplungsstücks Metall ist, wobei der eine oder die mehreren Vorsprünge des ersten Kopplungsstücks im Wesentlichen dreieckige Zähne sind, wobei die Bandöffnung des ersten Kopplungsstücks einen im Wesentlichen rechteckigen Querschnitt beinhaltet und wobei die Zähne in den im Wesentlichen rechteckigen Querschnitt vorstehen.

6. Schutzbrille (100) gemäß Anspruch 1, wobei der erste und der zweite Gehäusekörper (112A, 112B) jeweils gefaltetes Metallblech beinhalten.

7. Schutzbrille (100) gemäß Anspruch 1, die ferner Folgendes beinhaltet:
eine Schutzbrillenlinse (104), die innerhalb des Schutzbrillenrahmens (102) angeordnet ist.

8. Ein Verfahren zum Herstellen der Schutzbrille (100) gemäß Anspruch 1, wobei das Verfahren Folgendes beinhaltet:
Bilden des Schutzbrillenrahmens (102);
Bilden des ersten Kopplungsstücks (110) und des zweiten Kopplungsstücks (110),
wobei das Bilden des ersten Kopplungsstücks (110) das Bilden des ersten Gehäusekörpers (112A) und des zweiten Gehäusekörpers (112B) und das Koppeln des ersten Gehäusekörpers (112A) an den zweiten Gehäusekörper (112B) umfasst;
Koppeln des ersten Kopplungsstücks (110) an das erste Ende des elastischen Bands (108) und Koppeln des zweiten Kopplungsstücks (110) an das zweite Ende des elastischen Bands (108);
Einführen des ersten Kopplungsstücks (110) und des ersten Endes des elastischen Bands (108) in die erste Auslegeröffnung (122); und
Einführen des zweiten Kopplungsstücks (110) und des zweiten Endes des elastischen Bands (108) in die zweite Auslegeröffnung (122).

9. Verfahren gemäß Anspruch 8, wobei der erste Ausleger (106) und der zweite Ausleger (106) mit dem Schutzbrillenrahmen (102) gebildet werden.

10. Verfahren gemäß Anspruch 8, das ferner Folgendes beinhaltet:
Bilden des ersten Auslegers (106);
Bilden des zweiten Auslegers (106); und
Koppeln des ersten Auslegers (106) und des zweiten Auslegers (106) an den Schutzbrillenrahmen (102).

11. Verfahren gemäß Anspruch 8, wobei das Koppeln des ersten Kopplungsstücks (110) an das erste Ende des elastischen Bands (108) das Anordnen des ersten Endes des elastischen Bands (108) innerhalb der Bandöffnung (114) des ersten Kopplungsstücks (110) beinhaltet.

12. Verfahren gemäß Anspruch 8,
wobei das Bilden des Körpers (112) des ersten Kopplungsstücks (110) das Bilden des einen oder der mehreren Vorsprünge (116) innerhalb der Bandöffnung (114) beinhaltet.

13. Verfahren gemäß Anspruch 12, wobei der Körper (112) und der eine oder die mehreren Vorsprünge (116) des ersten Kopplungsstücks (110) durch maschinelles Bearbeiten eines Metallmaterials oder durch dreidimensionales Drucken gebildet werden.

14. Verfahren gemäß Anspruch 12, wobei das Bilden des Körpers (112) des ersten Kopplungsstücks (110) das Bilden des Körpers (112) aus Metallblech beinhaltet, um die Bandöffnung (114) zu definieren, und wobei das Bilden des einen oder der mehreren Vorsprünge (116) das Biegen der Vorsprünge (116) in die Bandöffnung (114) beinhaltet.

## Revendications

1. Un masque de protection (100) comprenant :
une bande élastique (108) ayant une première extrémité maintenue par une ou plusieurs saillies (116) à l'intérieur d'une ouverture pour bande (114) d'un corps (112) d'une première pièce d'accouplement (110) et une deuxième extrémité maintenue par une ou plusieurs saillies (116) à l'intérieur d'une ouverture pour bande (114) d'un corps (112) d'une deuxième pièce d'accouplement (110) ; et
une monture (102) de masque de protection ayant un premier stabilisateur (106) et un deuxième stabilisateur (106) disposés sur des extrémités latérales respectives de la monture (102), où les premier et deuxième stabilisateurs (106) ont chacun une ouverture pour stabilisateur (122) respective configurée pour recevoir l'une parmi les première et deuxième pièces d'accouplement (110) ;
où chacune des première et deuxième pièces d'accouplement (110) a un ou plusieurs crans (120) et chacune des première et deuxième ouvertures pour stabilisateur (122) a un ou plusieurs éléments correspondants (124) configurés pour s'interfacer avec les un ou plusieurs crans (120) ;
où chacune des première et deuxième pièces d'accouplement (110) est adaptée pour être insérée dans une ouverture respective des ouvertures pour stabilisateur (122) tout en maintenant la bande élastique (108), et pour être maintenue en place par les un ou plusieurs crans (120), de telle sorte que la bande élastique (108) est accouplée à la monture (102) de masque de protection ; et
où le corps (112) de la première pièce d'accouplement (110) comprend un premier corps de cage (112A) et un deuxième corps de cage (112B) accouplé au premier corps de cage (112A), et la bande élastique (108) est disposée entre les premier et deuxième corps de cage (112A, 112B).

2. Le masque de protection (100) de la revendication 1, où chacune des première et deuxième pièces d'accouplement (110) comprend en outre une première ouverture pour attache (118A-118E), où chaque première ouverture pour attache (118A-118E) est configurée pour recevoir une première attache (128A-128E).

3. Le masque de protection (100) de la revendication 2, où chacune des première et deuxième pièces d'accouplement (110) comprend en outre une deuxième ouverture pour attache (118A-118E), où chaque deuxième ouverture pour attache (118A-E) est configurée pour recevoir une deuxième attache (128A-128E), et où les première et deuxième ouvertures pour attache sont disposées sur des côtés opposés de la pièce d'accouplement (110).

4. Le masque de protection (100) de la revendication 1, où les un ou plusieurs crans (120) sont des saillies et les éléments correspondants (124) sont des creux.

5. Le masque de protection (100) de la revendication 1, où le corps de la première pièce d'accouplement est en métal, où les une ou plusieurs saillies de la première pièce d'accouplement sont des dents de forme substantiellement triangulaire, où l'ouverture pour bande de la première pièce d'accouplement comprend une section transversale substantiellement rectangulaire, et où les dents font saillie jusque dans la section transversale substantiellement rectangulaire.

6. Le masque de protection (100) de la revendication 1, où les premier et deuxième corps de cage (112A, 112B) comprennent chacun une tôle métallique pliée.

7. Le masque de protection (100) de la revendication 1, comprenant en outre :
une lentille de masque de protection (104) disposée à l'intérieur de la monture (102) de masque de protection.

8. Un procédé de fabrication du masque de protection (100) de la revendication 1, le procédé comprenant :
le formage de la monture (102) de masque de protection ;
le formage de la première pièce d'accouplement (110) et de la deuxième pièce d'accouplement (110), où le formage de la première pièce d'accouplement (110) inclut le formage du premier corps de cage (112A) et du deuxième corps de cage (112B) et l'accouplement du premier corps de cage (112A) au deuxième corps de cage (112B) ;
l'accouplement de la première pièce d'accouplement (110) à la première extrémité de la bande élastique (108), et l'accouplement de la deuxième pièce d'accouplement (110) à la deuxième extrémité de la bande élastique (108) ;
l'insertion de la première pièce d'accouplement (110) et de la première extrémité de la bande élastique (108) à l'intérieur de la première ouverture pour stabilisateur (122) ; et
l'insertion de la deuxième pièce d'accouplement (110) et de la deuxième extrémité de la bande élastique (108) à l'intérieur de la deuxième ouverture pour stabilisateur (122).

9. Le procédé de la revendication 8, où le premier stabilisateur (106) et le deuxième stabilisateur (106) sont formés avec la monture (102) de masque de protection.

10. Le procédé de la revendication 8, comprenant en outre :
le formage du premier stabilisateur (106) ;
le formage du deuxième stabilisateur (106) ; et
l'accouplement du premier stabilisateur (106) et du deuxième stabilisateur (106) à la monture (102) de masque de protection.

11. Le procédé de la revendication 8, où l'accouplement de la première pièce d'accouplement (110) à la première extrémité de la bande élastique (108) comprend la disposition de la première extrémité de la bande élastique (108) à l'intérieur de l'ouverture pour bande (114) de la première pièce d'accouplement (110).

12. Le procédé de la revendication 8,
où le formage du corps (112) de la première pièce d'accouplement (110) comprend le formage des une ou plusieurs saillies (116) à l'intérieur de l'ouverture pour bande (114).

13. Le procédé de la revendication 12, où le corps (112) et les une ou plusieurs saillies (116) de la première pièce d'accouplement (110) sont formés par usinage d'un brut métallique ou impression tridimensionnelle.

14. Le procédé de la revendication 12, où le formage du corps (112) de la première pièce d'accouplement (110) comprend le formage du corps (112) à partir d'une tôle métallique pour définir l'ouverture pour bande (114), et où le formage des une ou plusieurs saillies (116) comprend le rabattement des saillies (116) jusque dans l'ouverture pour bande (114).
